# EUROPEAN PATENT SPECIFICATION

(11) **EP 2 732 840 B1**
(45) Date of publication and mention of the grant of the patent: **27.05.2015**
(21) Application number: 12192578.8
(22) Date of filing: 14.11.2012
(51) Int. Cl.: A61M 16/08, A61M 16/20

(54) **Device for controlled supply of a medical gas**
Vorrichtung für die gesteuerte Zufuhr eines medizinischen Gases
Dispositif d'alimentation contrôlée de gaz médical

(43) Date of publication of application: 21.05.2014
(73) Proprietor: Flow Meter S.p.a., 24040 Levate (Bergamo) (IT)
(72) Inventor: Paratico, Roberto, 24040 Levate (Bergamo) (IT)
(74) Representative: Botti, Mario

(56) References cited:
- WO-A2-2006/133175
- US-A1- 2003 038 479
- US-A1- 2010 122 734
- US-B1- 7 617 826

## Description

### Field of application

The present invention relates to a device for controlled supply of a medical gas, in particular of the type designed to regulate and/or measure a gas flow from a distribution network or from a pressure vessel and supply it to one or more user appliances.

### Prior Art

In various applications in different technological fields it is required to perform the regulation and/or measurement of the flowrate and/or of the pressure of a gas or of a generic fluid flowing in circuits or pipes.

In particular, in the medical sector regulators and measuring devices of various kinds are used to dose the supply of pressurised gas intended for therapeutic applications, such as oxygen therapy.

These devices may be applied to the outlet of a distribution network or, if equipped with a suitable high-pressure reducer which is integrated or connected in series, they may be applied to a pressurised vessel containing medical gas.

They therefore comprise a flow inlet, which is connected to the medical gas source, and a flow path which leads to one or more outlets.

The flow path, depending on the type of device, may be provided with one or more pressure reduction stages, an optional pressure gauge designed to read the pressure upstream of the device, an optional flowmeter for reading the flowrate downstream of the device, and a flowrate regulator of the type with calibrated orifices or needle valve.

A threaded fitting which allows connection of a user appliance is also provided at the flow path outlet.

Document US 2003/0038479 discloses a device for sealingly connecting together a pair of fluid-conditioning units, wherein a threaded fitting connects together two opposite side apertures of the fluid-conditioning units.

The devices of the type described above, although substantially satisfying the requirements of the sector, nevertheless have a number of drawbacks which hitherto have not been overcome.

The main drawback consists in the specific nature of the threaded connector provided at the outlet. In the medical sector, in fact, a single set of regulations defining a single globally recognised standard for threaded connections does not exist. Instead, there exist a series of national regulations which propose standards valid for a particular country, together with other type-approval certifications which categorize similar appliances, such as the standard governing pre-filled humidification systems.

In order to meet the demand of the international market, the manufacturer must therefore provide a series of models which differ solely in terms of the type of outlet fitting, with a substantial increase in the production and warehouse costs.

Moreover, the limited flexibility of use of the device constitutes a drawback also for end users. The latter in fact may connect only certain categories of appliances to the outlet of their device and must modify the entire installation if they wish to connect to the same outlet of the distribution network an appliance fitted with a non-compatible connector.

The technical problem forming the basis of the present invention is therefore that of devising a supply device which has structural and functional characteristics such as to overcome the drawback mentioned above in connection with the prior art and which is also easy to use for healthcare staff and has a low manufacturing cost.

### Summary of the invention

The aforementioned technical problem is solved by a device for controlled supply of a medical gas as defined in claim 1.

As will be evident to a person skilled in the art, the possibility of releasing externally the fastening means allows operating staff without particular skills to replace the threaded fitting quickly, thus adapting the supply device.

The threaded fitting has, in particular, the first threaded connector at one of its ends and a second threaded connector at the opposite end, said threaded fitting being able to be alternately positioned in two operating configurations where the threaded fitting has, respectively, the first or second threaded connectors directed downstream so as to define the outlet of the flow supply path, release of said fastening means allowing the operating configuration of said threaded fitting to be modified.

The advantage associated with having two different connectors included inside the same device, is evident. The operating staff, without having to use other parts external to the device, may thus rapidly modify the fitting when needed.

Said first and second threaded connectors advantageously comply with two different standards, namely have different diameter sizes and/or thread parameters.

The device may also comprise at least one alternative threaded fitting which may replace said threaded fitting and which comprises at one of its ends at least one third threaded connector complying with a standard different from the first two standards.

The threaded fitting may be partially inserted inside a seat of the main body communicating with the flow supply path, said fastening means comprising at least one pin which can be selectively inserted inside a passage, preferably in the form of a slit, which is open on the outside of said main body and intercepts said seat, insertion of said pin inside said passage locking said threaded fitting inside said seat.

This locking system allows easy and rapid removal of the threaded fitting and also has the advantage that it is easy and inexpensive to produce.

Said pin may in particular be designed to be inserted inside a groove of the said threaded fitting.

Said pin may have two arms designed to be inserted on the two sides of said groove.

Said groove may consist of an annular recess provided in a central body from which the first threaded connector of the threaded fitting extends.

Said seat may be partially formed in a projecting sleeve of the main body, said passage being formed in the said projecting sleeve.

The device according to the invention may comprise means for controlling and/or measuring the medical gas flow supplied at the outlet.

In particular, it may comprise: at least one pressure reduction stage and a following flow regulation stage; a stop flow stage with a slide valve arranged so as to intercept said the flow supply path.

Said flow regulation stage may comprise a rotating drum provided with a plurality of calibrated orifices designed to intercept selectively the flow supply path.

Said flow regulation stage may comprise a flow regulator provided with a valve which allows continuous variation of the flowrate, said device also comprising a variable area flowmeter downstream of said stage.

The device may also comprise two or more following pressure reduction stages and a pressure gauge arranged upstream of the first of said stages.

Further characteristic features and advantages of the device for supply of medical gas according to the invention will emerge from the description, provided hereinbelow, of a number of examples of embodiment, provided by way of a non-limiting example, with reference to the accompanying drawings.

### Brief description of the drawings

Figure 1 shows a perspective view of a flow regulator according to the present invention;
Figure 2 shows a cross-sectional view, along a middle axis, of the flow regulator according to Figure 1;
Figure 3 shows an exploded perspective view of the flow regulator according to Figure 1;
Figure 4 shows a perspective view of a detail of the flow regulator according to Figure 1;
Figure 5 shows a perspective view of a flow regulator according to an alternative embodiment of the present invention;
Figure 6 shows an exploded perspective view of the flow regulator according to Figure 5;
Figure 7 shows a perspective view of a detail of the flow regulator according to Figure 5;
Figure 8 shows a perspective view of a pressure reducer according to an alternative embodiment of the present invention;
Figure 9 shows an exploded perspective view of the pressure regulator according to Figure 8;
Figure 10 shows a perspective view of a detail of the pressure regulator according to Figure 8.

### Detailed description

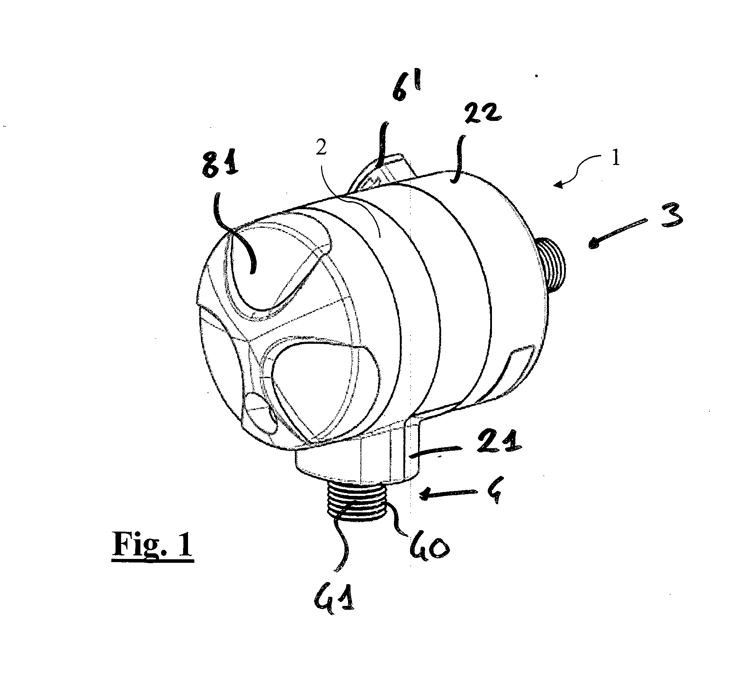

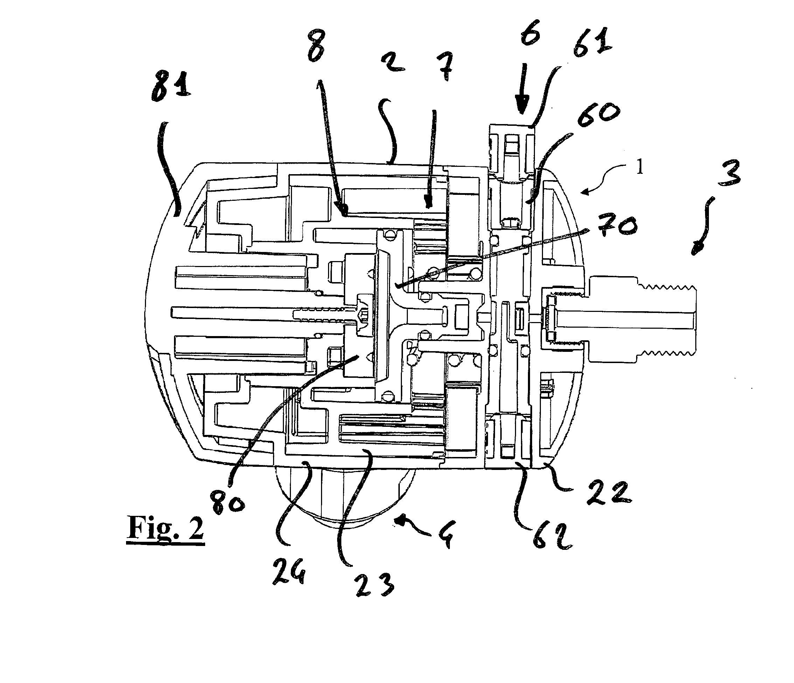

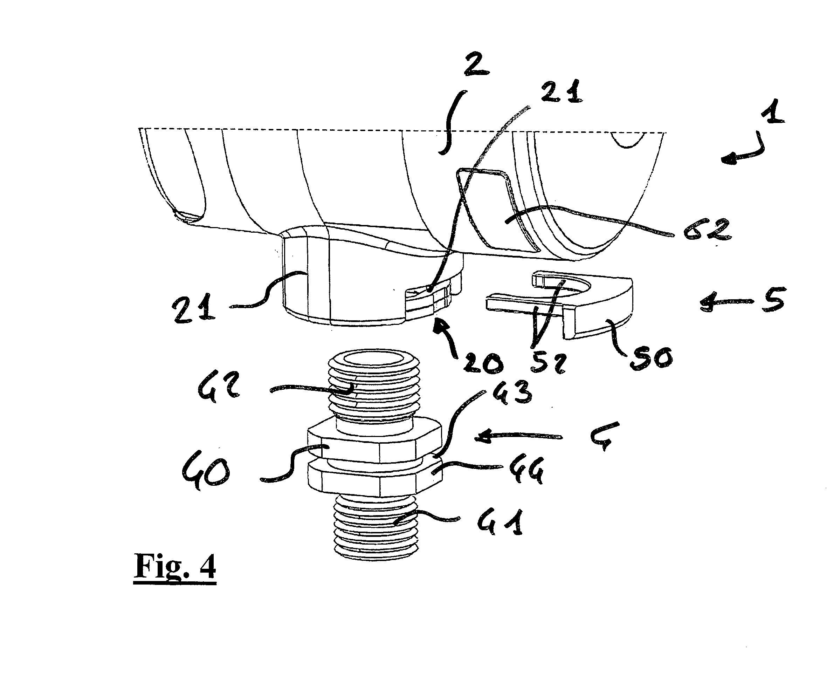

With reference to the accompanying Figures 1 to 4, 1 denotes in general a first embodiment of the device for supplying medical gas according to the present invention.

In this first embodiment, the device 1 is in the form of a flow regulator of the type with calibrated orifices.

The flow regulator comprises a main body 2, with a supporting and containment function, inside which at least one flow supply path extending between an inlet 3 - defined by a threaded connector of the known type - and an outlet 4 is formed.

The body 2 has a substantially cylindrical form coaxial with the threaded connector which defines the inlet 3; the outlet 3 instead has a radial orientation with respect to said main body 2.

The main body 2 may be made of metallic material, for example consisting of anodized aluminium or chrome-plated brass, but is preferably obtained by means of injection-moulding of polymer material, for example polycarbonate.

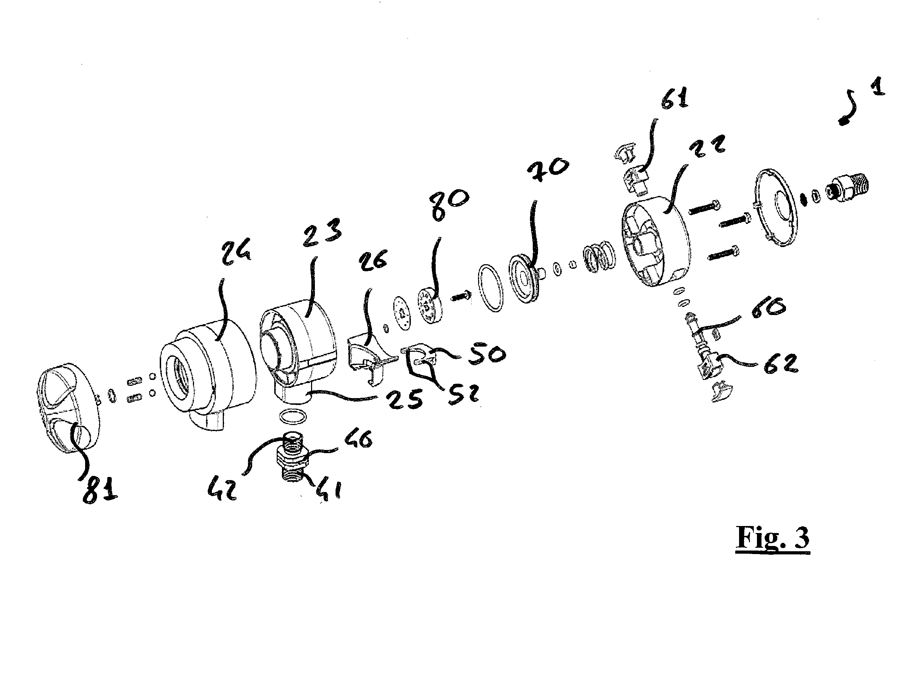

In fact, as can be seen in Figure 3, the main body 2 is composed of different portions obtained separately by means of moulding and joined together so as to define the internal and external form of the device.

Along the flow path there are arranged in series, in an upstream to downstream direction: a stop flow stage 6, a pressure reduction stage 7 and a flow regulation stage 8; these stages are all housed inside the main body 2.

In particular, the stop flow stage 6 is formed inside a first shell 22 of the main body 2 made as one piece by means of moulding.

Said first shell 22, with an essentially cylindrical outer form, is passed through transversely by a slide valve 60 which intercepts the flow supply path.

The slide valve 60 has a stem inserted inside a diametral hole of the first shell 22. This stem is movable between a closing position, in which it blocks the flow supply path, and an open position, in which said path is not blocked.

Two oppositely arranged pushbuttons 61, 62 are integral with the ends of the stem and project alternately from the cylindrical surface of the stop flow module depending on whether the stem is in the open or closed configuration.

It should be noted that the diametral hole which receives the stem has an offset axis, preferably inclined at 45°, with respect to the vertical middle plane along which the outlet 4 lies. This orientation of the slide valve 60 allows the overall dimensions to be advantageously reduced.

The main body also comprises a second inner shell 23 and a third outer shell 24 intended to house the first shell; both parts may be advantageously made by means of injection-moulding.

While the third outer shell 24 is substantially cup-shaped, the second shell 23 defines the internal form of the device.

In particular, it defines, together with the first shell 22 described above, an inner chamber which houses the pressure reduction stage 7.

The pressure reduction stage 7 comprises a compensation piston 70 sealingly slidable inside a cylindrical impression formed in the third shell 24 and pressed downstream by a compressed spring. The stem of the compensation piston 70 has an internal flow passage which is interrupted when the pressure downstream of the disc, in opposition to the thrust of the compressed spring, moves the stem into contact against a seat formed on the first shell 22. Otherwise the flow is free to pass through the stem and flow downstream of the disc.

The flow regulation stage 8 is also formed inside the second shell 23.

Said flow regulation stage comprises a rotating drum 80 provided with a plurality of calibrated orifices designed to intercept selectively the flow supply path.

This rotating drum 80, which is arranged along the longitudinal axis of the main body 2, is housed inside a cylindrical impression of the second shell 23 which opens out towards the pressure reduction stage 7.

Downstream of the rotating drum 80, the flow supply path follows an elbow-like path such that it emerges inside the radial outlet 4.

The rotating drum 80 is integrally joined to a regulation knob 81 which covers the bottom part of the cup-like third outer shell 23. The regulation knob 81 may be rotated by an operator and is designed to be locked, by means of snap-engagement, inside a plurality of positions corresponding to the calibrated orifices of the rotating drum 80.

The outlet 4 of the device opens out at the end of a sleeve 21 projecting radially from the main body 2. A seat 20 is formed inside said sleeve and has the function of housing a threaded fitting 40 of said outlet.

The threaded fitting 40 has a first threaded connector 41 at one of its ends and a second threaded connector 42 at the opposite end.

A non-threaded central part 44 is present between the two threaded connectors 41, 42 and has in particular a square chamfered-corner profile. The central part 44 has a groove 43 which is formed in particular by a recess which has an annular cross-section and the function of which will become clear below.

The seat 20 is partially formed inside an inner sleeve 25 formed as one piece with the second shell 23; said inner sleeve 25 is externally covered by the projecting sleeve 21 proper.

In particular the projecting sleeve is defined by two matching portions, one being integral with the third shell 24 and the other one being integral with a closing piece 26 which engages underneath the second shell 23.

It should be noted that the outer casing of the projecting sleeve 21, defined by the abovementioned structures, extends radially beyond the limit of the inner sleeve 25. An inner shoulder is thus defined inside the seat 20 and the central part 44 of the threaded fitting 40 bears against this shoulder.

In the operating configuration thus obtained, one of the threaded connectors 41, 42 is inserted inside the seat part 20 defined by the inner sleeve 25; the other connector instead projects beyond the projecting sleeve 21 so as to allow connection of a sanitary appliance.

The two threaded connectors 41, 42 described above comply with two different standards, namely have different diameter sizes and/or thread parameters. Thus, by reversing the orientation of the sleeve, it is possible to obtain two different operating configurations, depending on whether the first or second connector is to be positioned externally.

It should also be noted that it is possible to envisage the presence of other threaded connectors, not shown in the accompanying figures, so as to obtain further connection possibilities.

The device 1 obviously comprises fastening means 5 which allowed the sleeve to be locked in either one of its operating configurations.

These fastening means comprise a pin 50 with a flat C shape. The two arms 52 of the C are designed to be introduced into a slot-like passage 51 formed in the projecting sleeve portion 21 which extends beyond the inner sleeve 25; in particular, this passage is formed in the closing piece 26 defined above.

When the pin 50 is introduced into the passage 51 with the threaded fitting 40 arranged in an operating configuration, the two arms are inserted inside the groove 43, thus locking the threaded fitting 40 in position. In order to replace the threaded fitting 40 or reverse its orientation, the operator may easily remove the pin 50, thus releasing the connecting member.

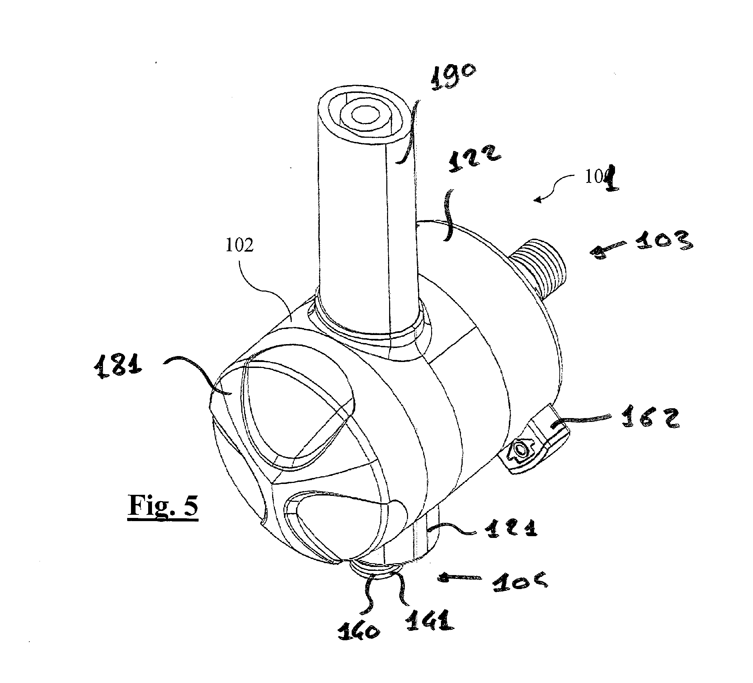

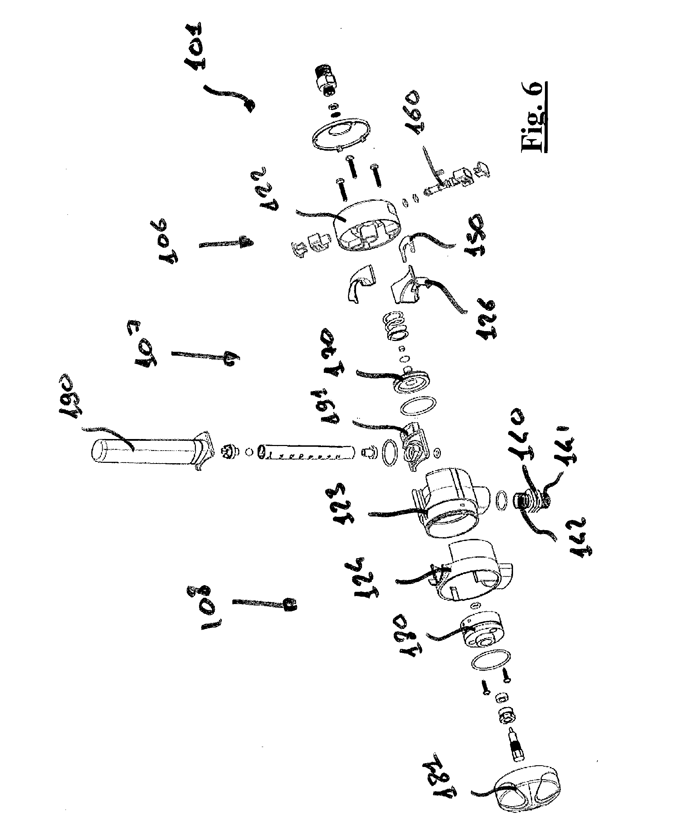

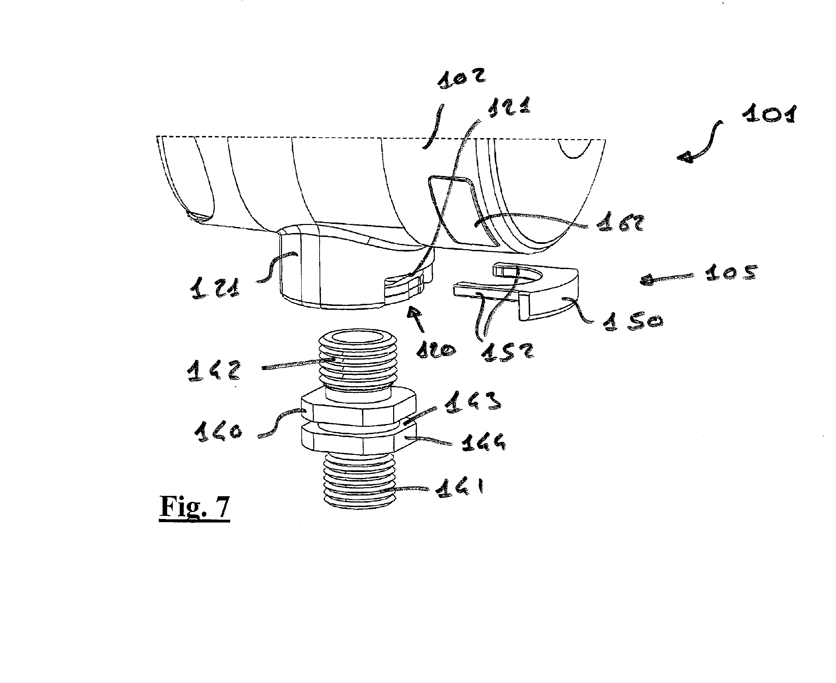

With reference to Figures 5 to 7, 101 denotes in general a second embodiment of the device for supplying medical gas according to the present invention.

In this second embodiment, the device 101 is in the form of a flow regulator with a needle valve, provided with a variable area flowmeter.

The description of said second embodiment focuses solely on the aspects which differ from those of the first embodiment. It should be noted that elements and parts, which are identical or similar to those of the first embodiment, are identified, both in the description and in the figures, by adding 100 to the identification number previously used.

The second embodiment differs principally from the first embodiment in that the flow regulation stage 108 comprises a valve 180 for continuous flow regulation, in particular a needle valve.

In view of the different nature of regulation, the regulation knob 181 may be no longer locked by means of snap-engagement in a plurality of separate positions. In order to allow locking of said regulation knob 181, it therefore has a locking mechanism of the push-pull (or push & lock) type.

Moreover, in order to allow reading of the selected flowrate in a continuous manner by means of the regulation knob, the devices has a column-type flowmeter 190 which is arranged upstream of the flow regulation stage 108 and supported by a specially provided support piece 191 which is slidably inserted above the second shell 123.

The second shell 123 and the third shell 124 have a form different from that of the first embodiment, intended to house the different regulating valve and support the column of the flowmeter 190.

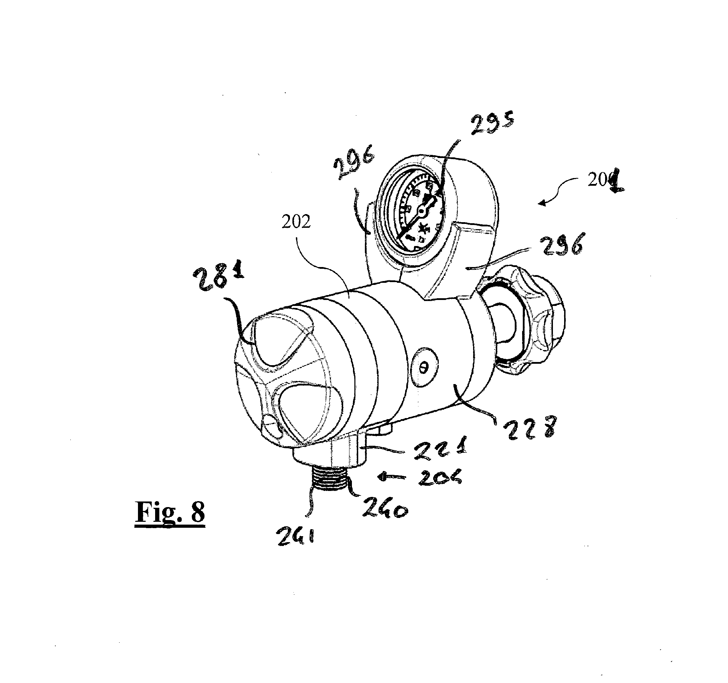

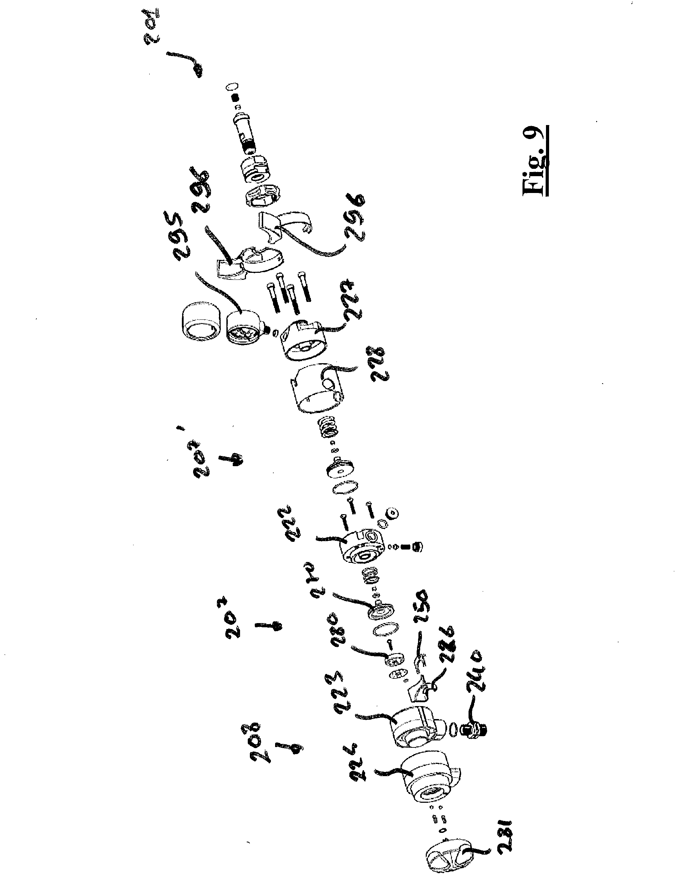

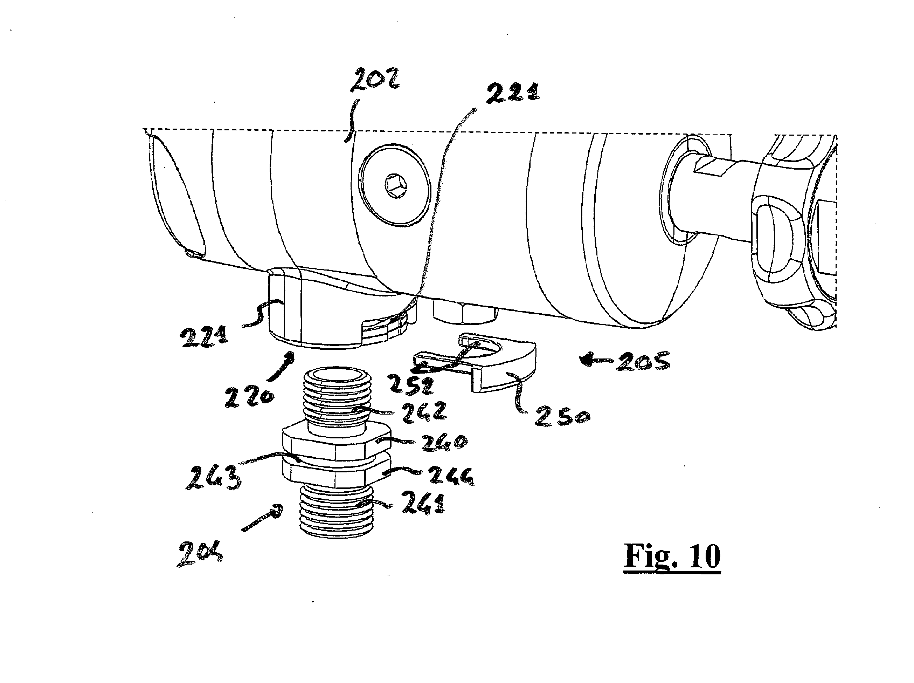

With reference to Figures 8 to 10, 201 denotes in general a third embodiment of the device for supplying medical gas according to the present invention.

In this second embodiment, the device 201 is in the form of a pressure reducer which incorporates a flow regulator with calibrated orifices.

Such a device 201 may be directly associated with a container for high-pressure medical gas, such as a gas cylinder, and is consequently supplied with an inlet fitting suitable for this purpose.

The description of said third embodiment focuses solely on the aspects which differ from those of the first embodiment. It should be noted that elements and parts, which are identical or similar to those of the first embodiment, are identified, both in the description and in the figures, by adding 200 to the identification number previously used.

The third embodiment differs principally from the first embodiment in that it comprises a pre-stage for reducing the pressure 207' which replaces the stop flow stage 6 described above.

The pressure reduction pre-stage 207' is in the form of a piston mechanism which is substantially similar to that of the main stage; said mechanism is housed between the first shell 222, suitably modified owing to the different function, and a fourth shell 227 which is associated upstream of the latter. The two shells have a reinforcing sleeve 228, preferably made of metallic material, arranged over them.

A pressure gauge 295 is provided upstream of the pressure reduction pre-stage 207', for measuring the pressure inside the container with which the device 201 is associated.

The reading dial of said pressure gauge is arranged on top of the fourth shell 227, in a position slightly inclined with respect to the vertical, in order to facilitate reading. Two lateral protection pieces 296 are provided in order to protect the dial from knocks, these pieces connecting the latter to the main body 202.

Obviously, a person skilled in the art, in order to satisfy any specific requirements which arise, may make numerous modifications and variations to the flow regulator, all of which being moreover contained within the scope of protection of the invention, as defined by the following claims.

## Claims

1. Device (1; 101; 201) for controlled supply of a medical gas, comprising a main body (2; 102; 202) which has, defined therein, at least one flow supply path extending between an inlet (3; 103; 203) and an outlet (4; 104; 204); and a threaded fitting (40; 140; 240) for connection to a user appliance; said threaded fitting (40; 140; 240) communicating with the flow supply path and having at least one first threaded connector (41; 141; 241) defining the outlet (4; 104; 204) of said path, said device (1; 101; 201) further comprising means (5; 105; 205) for fastening the threaded fitting (40; 140; 240) to the main body (2; 102; 202), which can be released from the outside of the main body (2; 102; 202) so as to allow removal of said threaded fitting (40; 140; 240); wherein said threaded fitting (40; 140; 240) has the first threaded connector (41; 141; 241) at one of its ends and a second threaded connector (42; 142; 242) at the opposite end; said threaded fitting (40; 140; 240) being able to be alternately positioned in two operating configurations where the threaded fitting (40' 140; 240) has respectively the first threaded connector (41; 141; 241) or the second threaded connector (42; 142; 242) directed downstream so as to define the outlet (4; 104; 204) of the flow supply path, release of said fastening means (5; 105; 205) allowing the operating configuration of said threaded fitting (40; 140; 240) to be modified; **characterized in that** said first threaded connector (41; 141; 241) and second threaded connector (42; 142; 242) comply with two different standards, namely have different diameter sizes and/or thread parameters.

2. Device (1; 101; 201) according to Claim 1, also comprising at least one alternative threaded fitting which may replace said threaded fitting (40; 140; 240) and which comprises at one of its ends at least one third threaded connector complying with a standard different from the first two standards.

3. Device (1; 101; 201) according to one of the preceding claims, wherein said threaded fitting (40; 140; 240) is partially inserted inside a seat (20; 120; 220) of the main body (2; 102; 202) communicating with the flow supply path, said fastening means (5; 105; 205) comprising at least one pin (50; 150; 250) which can be selectively inserted inside a passage (51; 151; 251) which opens out on the outside of said main body (2; 102; 202) and intercepts said seat (20; 120; 220), the insertion of said pin (50; 150; 250) inside said passage (51; 151; 251) locking said threaded fitting (40; 140; 240) inside said seat (20; 120; 220).

4. Device (1; 101; 201) according to Claim 3, wherein said pin (50; 150; 250) is designed to be inserted inside a groove (43; 143; 243) of said threaded fitting (40; 140; 240).

5. Device (1; 101; 201) according to Claim 4, wherein said pin (50; 150; 250) has two arms (52; 152; 252) designed to be inserted along the two sides of said groove (43; 143; 243).

6. Device (1; 101; 201) according to one of Claims 4 or 5, wherein said groove (43; 143; 243) is an annular recess provided in a central body (44; 144; 244) from which the first threaded connector (41; 141; 241) of the threaded fitting (40; 140; 240) extends.

7. Device (1; 101; 201) according to one of Claims 3 to 6, wherein said seat (20; 120; 220) is partially formed in a projecting sleeve (21; 121; 221) of the main body (2; 102; 202), said passage (51; 151; 251) being formed on said projecting sleeve (21; 121; 221).

8. Device (1; 101; 201) according to one of the preceding claims, also comprising means for controlling and/or measuring the medical gas flow supplied at the outlet (4; 104; 204).

9. Device (1; 101; 201) according to Claim 8, comprising at least one pressure reduction stage (7; 107; 207, 207') and a following flow regulation stage (8; 108; 208).

10. Device (1; 101) according to Claim 9, also comprising a stop flow stage (6; 106) with a slide valve (60, 160) arranged so as to intercept said flow supply path.

11. Device (1; 201) according to one of Claims 8 to 10, wherein said flow regulation stage (8; 208) comprises a rotating drum (80; 280) provided with a plurality of calibrated orifices designed to selectively intercept the flow supply path.

12. Device (101) according to one of Claims 8 to 10, wherein said flow regulation stage (108) comprises a valve which allows continuous variation of the flowrate, said device also comprising a variable area flowmeter (190) downstream of said stage.

13. Device (201) according to Claim 11, comprising two following pressure reduction stages (207, 207') and a pressure gauge (295) arranged upstream of the first of said stages (207).

## Patentansprüche

1. Vorrichtung (1; 101; 201) für eine gesteuerte Zufuhr eines medizinischen Gases, umfassend einen Hauptkörper (2; 102; 202), der wenigstens einen darin definierten Strömungszufuhrbahn aufweist, die sich zwischen einem Einlass (3; 103; 203) und einem Auslass (4; 104; 204) erstreckt, und einen Gewindestutzen (40; 140; 240) zur Verbindung mit einem Benutzergerät, besagter Gewindestutzen (40; 140; 240) steht in Kommunikation mit der Strömungszufuhrbahn und hat wenigstens ein erstes Gewindeverbindungsstück (41; 141, 241), das den Auslass (4; 104; 204) der genannten Bahn definiert,
die Vorrichtung (1; 101; 201) umfasst weiter Mittel (5; 105; 205) zur Befestigung des Gewindestutzens (40; 140; 240) an dem Hauptkörper (2; 102; 202), die von außerhalb des Hauptkörpers (2; 102; 202) gelöst werden können, um ein Entfernen des besagten Gewindestutzens (40; 140; 240) zu erlauben, wobei der besagte Gewindestutzen (40; 140; 240) das erste Gewindeverbindungsstück (41; 141; 241) an einem seiner Enden, und ein zweites Gewindeverbindungsstück (42; 142; 242) an dem gegenüberliegenden Ende aufweist;
besagter Gewindestutzen (40; 140; 240) ist alternativ in zwei Betriebskonfigurationen positionierbar, in denen der Gewindestutzen (40; 140; 240) entsprechend entweder das erste Gewindeverbindungsstück (41; 141, 241) oder das zweite Gewindeverbindungsstück (42; 142; 242) stromabwärts gerichtet hat, und so den Auslass (4; 104; 204) der Strömungszufuhrbahn definiert,
Freigabe der Mittel (5; 105; 205) zur Befestigung erlaubt es die Betriebskonfiguration des besagten Gewindestutzens (40; 140; 240) zu modifizieren; **dadurch gekennzeichnet, dass**
besagtes erstes Gewindeverbindungsstück (41; 141; 241) und zweites Gewindeverbindungsstück (42; 142; 242) zwei verschiedenen Standarts entsprechen, nämlich unterschiedliche Durchmessergrößen und/oder Gewindeparametern aufweisen.

2. Vorrichtung (1; 101; 201) gemäß Anspruch 1; weiter umfassend wenigstens einen alternativen Gewindestutzen, der besagten Gewindestutzen (40; 140; 240) ersetzen kann und der an einem seiner Enden wenigstens ein drittes Gewindeverbindungsstück aufweist, das einem zu den ersten beiden unterschiedlichen Standart entspricht.

3. Vorrichtung (1; 101; 201) gemäß einem der vorhergehenden Ansprüche, wobei besagter Gewindestutzen (40; 140; 240) teilweise in einem Sitz (20; 120; 220) des Hauptkörpers (2, 102; 202) kommunizierend mit der Strömungszufuhrbahn integriert ist, besagte Mittel zur Befestigung (5; 105; 205) umfassen wenigstens einen Pin (50; 150; 250), der selektiv in eine Passage (51; 151; 251) eingeführt werden kann, die sich außerhalb des besagten Hauptkörpers (2; 102; 202) öffnet und besagten Sitz (20; 120; 220) auffängt, das Einführen des besagten Pins (50; 150; 250) in besagte Passage (51; 151; 251) verriegelt besagten Gewindestutzen (40, 140; 240) in dem besagten Sitz (20; 120; 220).

4. Vorrichtung (1; 101; 201) gemäß Anspruch 3, wobei besagter Pin (50; 150; 250) dazu ausgebildet ist in einen Nut (43; 143; 243) des besagten Gewindestutzen (40; 140; 240) eingeführt zu werden.

5. Vorrichtung (1; 101; 201) gemäß Anspruch 4; wobei besagter Pin (50; 150; 250) zwei Arme (52; 152; 252) aufweist, die dazu ausgebildet sind entlang der zwei Seiten besagten Nut (43; 143; 243) eingeführt zu werden.

6. Vorrichtung (1; 101; 201) gemäß einem der Ansprüche 4 oder 5, wobei besagte Nut (43; 143; 243) eine ringförmige Ausnehmung in einem zentralen Körper (44; 144; 244) ist, von dem sich das erste Gewindeverbindungsstück (41; 141; 241) des Gewindestutzens (40; 140; 240) erstreckt.

7. Vorrichtung (1; 101; 201) gemäß einem der Ansprüche 3 bis 6, wobei besagter Sitz (20; 120; 220) teilweise in einer vorstehenden Muffe (21; 121; 221) des Hauptkörpers (2; 102; 202) ausgebildet ist, besagte Passage (51; 151; 251) ist an der besagten Muffe (21; 121; 221) ausgebildet.

8. Vorrichtung (1; 101; 201) gemäß einem der vorhergehenden Ansprüche, weiter umfassend Mittel zur Kontrolle und/oder Messung des medizinischen Gasstroms der am Auslass (4; 104; 204) geliefert wird.

9. Vorrichtung (1; 101; 201) gemäß Anspruch 8; umfassend wenigstens eine Druckreduzierstufe (7; 107; 207; 207') und eine nachgeschaltete Durchflussregelstufe (8; 108; 208).

10. Vorrichtung (1; 101) gemäß Anspruch 9, weiter umfassend eine Durchflussstopstufe (6; 106) mit einem Schieberventil (60; 160), das derart angeordnet, um die Strömungszufuhrbahn abzufangen.

11. Vorrichtung (1; 201) gemäß einem der Ansprüche 8 bis 10, wobei besagte Durchflussregelstufe (8; 208) eine Drehtrommel (80; 280) umfasst, die mit einer Vielzahl von kalibrierten Öffnungen versehen ist, um selektiv die Strömungszufuhrbahn abzufangen.

12. Vorrichtung (101) gemäß einem der Ansprüche 8 bis 10, wobei besagte Durchflussregelstufe (108) ein Ventil umfasst, das eine kontinuierliche Regulierung der Durchflussrate erlaubt, besagtes Bauteil weiter einen Durchflussmesser (190) für variable Bereiche stromabwärts der besagten Stufe umfasst.

13. Vorrichtung (201) gemäß Anspruch 11, umfassend zwei aufeinander folgende Druckreduzierstufen (207; 207') und ein Manometer (295), das stromaufwärts von der ersten der genannten Stufe (207) angeordnet ist.

## Revendications

1. Dispositif (1 ; 101 ; 201) d'alimentation contrôlée en un gaz médical, comprenant un corps principal (2 ; 102 ; 202) qui comporte, défini dans celui-ci, au moins un chemin de flux d'alimentation s'étendant entre une entrée (3 ; 103 ; 203) et une sortie (4 ; 104 ; 204) ; et un raccord fileté (40 ; 140 ; 240) pour raccordement à un appareil utilisateur; ledit raccord fileté (40 ; 140 ; 240) communiquant avec le chemin de flux d'alimentation et comportant au moins un premier connecteur fileté (41 ; 141 ; 241) définissant la sortie (4 ; 104 ; 204) dudit chemin, ledit dispositif (1 ; 101 ; 201) comprenant en outre des moyens (5 ; 105 ; 205) pour fixer le raccord fileté (40 ; 140 ; 240) au corps principal (2 ; 102 ; 202), qui peuvent être libérés de l'extérieur du corps principal (2 ; 102 ; 202) de manière à permettre le retrait dudit raccord fileté (40 ; 140 ; 240) ; dans lequel ledit raccord fileté (40 ; 140 ; 240) comporte le premier connecteur fileté (41 ; 141 ; 241) à l'une de ses extrémités et un deuxième connecteur fileté (42 ; 142 ; 242) à l'extrémité opposée ; ledit raccord fileté (40 ; 140 ; 240) pouvant être placé de manière alternée dans deux configurations de fonctionnement où le raccord fileté (40 ; 140 ; 240) comporte respectivement le premier connecteur fileté (41 ; 141 ; 241) ou le deuxième connecteur fileté (42 ; 142 ; 242) orienté dans le sens aval de manière à définir la sortie (4 ; 104 ; 204) du chemin de flux d'alimentation, la libération desdits moyens de fixation (5 ; 105 ; 205) permettant à la configuration de fonctionnement dudit raccord fileté (40 ; 140 ; 240) d'être modifiée ; **caractérisé en ce que** lesdits premier connecteur fileté (41 ; 141 ; 241) et deuxième connecteur fileté (42 ; 142 ; 242) sont conformes à deux normes différentes, à savoir possèdent des diamètres et/ou des paramètres de filetage différents.

2. Dispositif (1 ; 101 ; 201) selon la revendication 1, comprenant également au moins un autre raccord fileté qui peut remplacer ledit raccord fileté (40 ; 140 ; 240) et qui comprend à l'une de ses extrémités au moins un troisième connecteur fileté conforme à une norme différente des deux premières normes.

3. Dispositif (1 ; 101 ; 201) selon l'une des revendications précédentes, dans lequel ledit raccord fileté (40 ; 140 ; 240) est introduit en partie à l'intérieur d'un siège (20 ; 120 ; 220) du corps principal (2 ; 102 ; 202) communiquant avec le chemin de flux d'alimentation, lesdits moyens de fixation (5 ; 105 ; 205) comprenant au moins un axe (50 ; 150 ; 250) qui peut être introduit sélectivement à l'intérieur d'un passage (51 ; 151 ; 251) qui ouvre sur l'extérieur dudit corps principal (2 ; 102 ; 202) et coupe ledit siège (20 ; 120 ; 220), l'introduction dudit axe (50 ; 150 ; 250) à l'intérieur dudit passage (51 ; 151 ; 251) bloquant ledit raccord fileté (40 ; 140 ; 240) à l'intérieur dudit siège (20 ; 120 ; 220).

4. Dispositif (1 ; 101 ; 201) selon la revendication 3, dans lequel ledit axe (50 ; 150 ; 250) est conçu pour être introduit à l'intérieur d'une rainure (43 ; 143 ; 243) dudit raccord fileté (40 ; 140 ; 240).

5. Dispositif (1 ; 101 ; 201) selon la revendication 4, dans lequel ledit axe (50 ; 150 ; 250) comporte deux bras (52 ; 152 ; 252) conçus pour être introduits le long des deux côtés de ladite rainure (43 ; 143 ; 243).

6. Dispositif (1 ; 101 ; 201) selon l'une des revendications 4 ou 5, dans lequel ladite rainure (43 ; 143 ; 243) est un évidement annulaire prévu dans un corps central (44 ; 144 ; 244) à partir duquel s'étend le premier connecteur fileté (41 ; 141 ; 241) du raccord fileté (40 ; 140 ; 240).

7. Dispositif (1 ; 101 ; 201) selon l'une quelconque des revendications 3 à 6, dans lequel ledit siège (20 ; 120 ; 220) est formé en partie dans un manchon en saillie (21 ; 121 ; 221) du corps principal (2 ; 102 ; 202), ledit passage (51 ; 151 ; 251) étant formé sur ledit manchon en saillie (21 ; 121 ; 221).

8. Dispositif (1 ; 101 ; 201) selon l'une des revendications précédentes, comprenant également des moyens de contrôle et/ou de mesure du flux de gaz médical fourni à la sortie (4 ; 104 ; 204).

9. Dispositif (1 ; 101 ; 201) selon la revendication 8, comprenant au moins un étage de réduction de pression (7 ; 107 ; 207, 207') et un étage suivant de régulation de débit (8 ; 108 ; 208).

10. Dispositif (1 ; 101) selon la revendication 9, comprenant également un étage d'arrêt d'écoulement (6 ; 106) avec un robinet à tiroir (60, 160) agencé de manière à couper ledit chemin de flux d'alimentation.

11. Dispositif (1 ; 201) selon l'une des revendications 8 à 10, dans lequel ledit étage de régulation de débit (8 ; 208) comprend un tambour rotatif (80 ; 280) pourvu d'une pluralité d'orifices calibrés conçus pour couper sélectivement le chemin de flux d'alimentation.

12. Dispositif (101) selon l'une des revendications 8 à 10, dans lequel ledit étage de régulation de débit (108) comprend une soupape qui permet une variation continue du débit, ledit dispositif comprenant également un débitmètre à section variable (190) en aval dudit étage.

13. Dispositif (201) selon la revendication 11, comprenant deux étages suivants de réduction de pression (207, 207') et un manomètre (295) agencé en amont du premier desdits étages (207).
